# EUROPEAN PATENT SPECIFICATION

(11) **EP 4 025 240 B1**
(45) Date of publication and mention of the grant of the patent: **30.10.2024**
(21) Application number: 20772502.9
(22) Date of filing: 02.09.2020
(51) Int. Cl.: A61K 38/20, A61K 38/57, A61K 9/00, A61K 45/06, A61K 47/68, A61P 1/00, A61P 1/12

(54) **IL-22 ORAL, INTRA-RECTAL, OR OTHER GUT-RELATED COMPOSITIONS AND METHODS OF USE THEREOF**
IL-22 ORALE, INTRA-REKTALE ODER ANDERE DARMBEZOGENE ZUSAMMENSETZUNGEN UND VERFAHREN ZUR VERWENDUNG DAVON
COMPOSITIONS D'IL-22 ORALES, INTRARECTALES, OU AUTRES COMPOSITIONS ASSOCIÉES À L'INTESTIN ET PROCÉDÉS D'UTILISATION DE CELLES-CI

(30) Priority: 03.09.2019 US 201962895179 P
(43) Date of publication of application: 13.07.2022
(73) Proprietor: Amgen Inc., Thousand Oaks, CA 91320-1799 (US)
(72) Inventor: HSU, Hailing, Thousand Oaks, California 91320-1799 (US)
(74) Representative: Lau, Sarah Jane
(86) International application number: PCT/US2020/049073
(87) International publication number: WO 2021/046133

(56) References cited:
- WO-A1-2016/128974
- AU-A1- 2014 201 310
- US-A1- 2018 355 009
- US-A1- 2019 119 407
- CAROLINE TWAROG ET AL: "Intestinal Permeation Enhancers for Oral Delivery of Macromolecules: A Comparison between Salcaprozate Sodium (SNAC) and Sodium Caprate (C10)", PHARMACEUTICS, vol. 11, no. 2, 13 February 2019 (2019-02-13), pages 78, XP055634457, DOI: 10.3390/pharmaceutics11020078
- WITTE K ET AL: "IL-28A, IL-28B, and IL-29: Promising cytokines with type I interferon-like properties", CYTOKINE AND GROWTH FACTOR REVIEWS, ELSEVIER LTD, GB, vol. 21, no. 4, 1 August 2010 (2010-08-01), pages 237 - 251, XP027267638, ISSN: 1359-6101, [retrieved on 20100723]

## Description

### FIELD OF THE INVENTION

The present invention relates to oral, intra-rectal, or other gut-related compositions comprising a protein, an absorption enhancer, optionally a protease inhibitor and a method for administering same. In particular, the present invention relates to oral, intra-rectal, or other gut-related compositions containing IL-22 or IL-22-Fc and uses in treatment of inflammatory disorders, such as irritable bowel disease (IBD).

### BACKGROUND OF THE INVENTION

Interleukin-22 (IL-22) is a class II cytokine that is up-regulated in T cells. One function of IL-22 is to enhance the innate immunity of peripheral tissues by inducing the expression of anti-microbial peptides (Wolk et al., Immunity, 21 :241-54,2004; Boniface et al., J. Immunol., 174:3695-3702,2005). Other studies have shown that expression ofIL-22 mRNA is induced in vivo in response to LPS administration, and that IL-22 modulates parameters indicative of an acute phase response (Dumoutier L. et al., Genes Immunol., 1 (8):488-494, (2000); Pittman et al., Genes and Immunity, 2:172, 2001). Taken together, these observations indicate that IL-22 plays a role in inflammation (Kotenko S. V., Cytokine & Growth Factor Reviews, 13(3):223-40, 2002). Several T cell disorders are associated with increased levels of IL-22 (Wolk et al., Immunity, 21:241-54,2004; Ikeuchi H. et al., Arthritis Rheum., 52: 1037-1046,2005); Andoh, A. et aI., Gastroenterology, 129:969-984,2005).

Currently there are limitations for administration of IL-22 for treatment of inflammatory diseases. The present invention addresses the need for alternative methods of administration of IL-22.
US 2018/355009 describes IL-22 Fc fusion protein and methods of use.

### SUMMARY OF THE INVENTION

Aspects of the invention for which protection is sought are defined in the appended set of claims. The description may contain additional technical information, which although not part of the claimed invention, is provided to place the invention in a broader technical context and to illustrate possible related technical developments.

In one aspect, the invention provides an oral, intra-rectal, or other gut-related pharmaceutical composition comprising an IL-22 protein and an absorption enhancer, wherein the absorption enhancer is N(8-[2-hydroxybenzoyl]amino)caprylic acid (NAC) or a salt of said NAC. In a further aspect, the invention provides the oral, intra-rectal, or other gut-related pharmaceutical composition as defined in the appended claims, for use in a method for treating an inflammatory disorder.

The present invention is based, in part, on an oral, intra-rectal, or other gut-related pharmaceutical composition comprising an IL-22 or IL-22-Fc protein and an absorption enhancer of N(8-[2-hydroxybenzoyl]amino)caprylic acid (NAC) or a salt of said NAC. In some embodiments, the IL-22 has at least 80% identity to SEQ ID NO: 1, 2, 3, or 4. In some embodiments, the salt of said NAC is selected from the group consisting of a monosodium salt, a disodium salt, and a combination thereof. In some embodiments, the composition further comprises a protease inhibitor, wherein said protease inhibitor is selected from the group consisting of a serpin, a suicide inhibitor, a transition state inhibitor, a protein protease inhibitor, a cheating agent, a cysteine protease inhibitor, a threonine protease inhibitor, an aspartic protease inhibitor, and a metalloprotease inhibitor. In some embodiments, the composition further comprises EDTA or a salt thereof, and/or a coating that inhibits digestion of said composition in a stomach of a subject; an emeric coating; or a gelatin coating.

In some embodiments, then invention is an Fc-fusion protein of any of the above compositions.

The invention provides the oral, intra-rectal, or other gut-related pharmaceutical composition as defined in the appended claims, or any of the Fc-fusion proteins as defined in the appended claims for use in a method for treating an inflammatory disorder.

In some embodiments, the invention includes an inflammatory disorder selected from inflammatory bowel disorder, Crohn's disease, ulcerative colitis, Type II diabetes, Type II diabetes with morbid obesity, wounds (including diabetic wounds and diabetic ulcers), burns, ulcers (including pressure ulcer and venous ulcer), graft versus host disease (GVHD), microbial infection, acute kidney injury, acute pancreatitis, wounds, cardiovascular conditions, metabolic syndrome, acute endotoxemia, sepsis, atherosclerosis, cardiovascular disease, metabolic syndrome, endotoxemia (acute and mild), sepsis, acute coronary heart disease, hypertension, dyslipemia, obesity, hyperglycemia, lipid metabolism disorders, hepatitis, acute hepatitis, renal failure, acute renal failure, acute kidney injury, renal draft failure, post cadaveric renal transplant delayed graft function, contrast induced nephropathy, pancreatitis, acute pancreatitis, liver fibrosis and lung fibrosis.

### BRIEF DESCRIPTION OF THE FIGURES

Figure 1 shows serum levels of (a) IL-22/SNAC mixture or (b) serum amyloid A.
Figure 2 shows serum levels of (a) IL-22/SNAC mixture or (b) serum amyloid A from individual mice.
Figure 3 shows levels of IL-22 retained in the colon versus systemic absorption from Fc-bound IL-22 (Figure 3a) and IL-22 alone (Figure 3b).
Figure 4 shows differences in systemic versus colonic IL-22 levels from intra-rectal (Figure 4a) and intra-peritoneal administration (Figure 4b).
Figure 5 shows serum amyloid acid (SAA) levels systemically or in the gut following intra-peritoneal and intra-rectal administration of murine Fc-IL-22. Figure 5a shows systemic SAA levels and Figure 5b shows gut SAA levels.

### DETAILED DESCRIPTION OF THE INVENTION

The references to the methods of treatment by therapy or surgery or in vivo diagnosis methods in this description are to be interpreted as references to compounds, pharmaceutical compositions and medicaments for use in those methods. The present invention is directed to compositions and methods for oral, intra-rectal, or other gut-related administration of IL-22. Throughout the application, IL-22 includes IL-22-Fc, an Fc-fusion protein. The invention provides IL-22 in an oral, intra-rectal, or other gut-related pharmaceutical composition with an absorption enhancer. In some embodiments, IL-22 may be in an oral, intra-rectal, or other gut-related pharmaceutical composition with a protease inhibitor and an absorption enhancer. In some embodiments, the absorption enhancer increases the absorption of IL-22 through an intestinal mucosal layer. In some embodiments, the present invention is a method of treating a patient having an inflammatory disorder by administering any of the oral, intra-rectal, or other gut-related pharmaceutical compositions described above and further detailed below. In some embodiments, the inflammatory disorder can be, for example, irritable bowel disease (IBD).

In another embodiment, the compositions of the present invention include IL-22, an absorption enhancer, and a carrier. In another embodiment, the compositions of the present invention include IL-22, a protease inhibitor, and a carrier. In another embodiment, the compositions of the present invention are used to deliver IL-22 through various biological, chemical, and physical barriers which can decrease degradation of IL-22. In another embodiment, the compositions comprise IL-22 and SNAC. In another embodiment, the compositions comprise IL-22, SNAC, a protease inhibitor, and EDTA or Na-EDTA. In another embodiment, the compositions comprise IL-22, SNAC, a protease inhibitor, and an omega-3 fatty acid. In another embodiment, the compositions comprise IL-22, SNAC, a protease inhibitor, EDTA or Na-EDTA, and an omega-3 fatty acid. In another embodiment, the composition is an oral, intra-rectal, or other gut-related pharmaceutical composition. In another embodiment, the composition as described herein is an encapsulated oral, intra-rectal, or other gut-related pharmaceutical composition. In another embodiment, the composition as described herein is in a liquid oral, intra-rectal, or other gut-related dosage form. In another embodiment, the composition as described herein is in a dry oral, intra-rectal, or other gut-related dosage form (tablet etc.).

Interleukin-22 (IL-22) is a member of the IL-10 family of cytokine that is produced by Th22 cells, NK cells, lymphoid tissue inducer (LTi) cells, dendritic cells and Thl7 cells. IL-22 binds to the IL-22R1/IL-10R2 receptor complex, which is expressed in innate cells such as epithelial cells, hepatocytes, and keratinocytes and in barrier epithelial tissues of several organs including dermis, pancreas, intestine and the respiratory system. IL-22 is known to regulate host defense and tissue regeneration and treatment with IL-22 was effective in treating dextran sulfate sodium (DSS)-induced colitis in mice.

In certain embodiments, the IL-22 polypeptide comprises the amino acid sequence of human IL-22 SEQ ID NO: 1 (precursor protein) or SEQ ID NO:2. In some embodiments, the IL-22 polypeptide comprises an amino acid sequence having 80% identity to SEQ ID NO: 1 or 2. In some embodiments, the IL-22 polypeptide comprises an amino acid sequence having 90% identity to SEQ ID NO: 1 or 2. In some embodiments, the IL-22 polypeptide comprises an amino acid sequence having 95% identity to SEQ ID NO: 1 or 2. In some embodiments, the IL-22 polypeptide comprises an amino acid sequence having 99% identity to SEQ ID NO: 1 or 2. Human IL-22 amino acid sequence of SEQ ID NO: 1 and 2 is as follows:
Sequence of human IL-22 precursor (Q9GZX6): 
MAALQKSVSSFLMGTLATSCLLLLALLVQGGAAAPISSHCRLDKSNFQQPYITNRTF
MLAKEASLADNNTDVRLIGEKLFHGVSMSERCYLMKQVLNFTLEEVLFPQSDRFQP
YMQEVVPFLARLSNRLSTCHIEGDDLHIQRNVQKLKDTVKKLGESGEIKAIGELDLLF
MSLRNACI (SEQ ID NO:1)
Sequence of human IL-22 (Q9GZX6):

APISSHCRLDKSNFQQPYITNRTFMLAKEASLADNNTDVRLIGEKLFHGVSMSERCYL
MKQVLNFTLEEVLFPQSDRFQPYMQEVVPFLARLSNRLSTCHIEGDDLHIQRNVQKL
KDTVKKLGESGEIKAIGELDLLFMSLRNACI (SEQ ID NO:2)

In certain embodiments, the IL-22 polypeptide comprises the amino acid sequence of mouse IL-22 SEQ ID NO:3 (precursor) or SEQ ID NO:4. In some embodiments, the IL-22 polypeptide comprises an amino acid sequence having 80% identity to SEQ ID NO:3 or 4. In some embodiments, the IL-22 polypeptide comprises an amino acid sequence having 90% identity to SEQ ID NO:3 or 4. In some embodiments, the IL-22 polypeptide comprises an amino acid sequence having 95% identity to SEQ ID NO:3 or 4. In some embodiments, the IL-22 polypeptide comprises an amino acid sequence having 99% identity to SEQ ID NO:3 or 4. Mouse IL-22 amino acid sequence of SEQ ID NO:3 and 4 is as follows:
Sequence of mouse IL-22 precursor: 
MAVLQKSMSFSLMGTLAASCLLLIALWAQEANALPVNTRCKLEVSNFQQPYIVNRT
FMLAKEASLADNNTDVRLIGEKLFRGVSAKDQCYLMKQVLNFTLEDVLLPQSDRFQ
PYMQEVVPFLTKLSNQLSSCHISGDDQNIQKNVRRLKETVKKLGESGEIKAIGELDLL
FMSLRNACV (SEQ ID NO:3)
Sequence of mouse IL-22:

ALPVNTRCKLEVSNFQQPYIVNRTFMLAKEASLADNNTDVRLIGEKLFRGVSAKDQC
YLMKQVLNFTLEDVLLPQSDRFQPYMQEVVPFLTKLSNQLSSCHISGDDQNIQKNVR
RLKETVKKLGESGEIKAIGELDLLFMSLRNACV (SEQ ID NO:4)

IL-22 plays an important role in mucosal immunity, mediating early host defense against attaching and effacing bacterial pathogens. See Zheng et al., 2008, Nat. Med. 14:282-89. IL-22 promotes the production of anti-microbial peptides and proinflammatory cytokines from epithelial cells and stimulates proliferation and migration of colonic epithelial cells in the gut. See Kumar et al., 2013, J. Cancer, 4:57-65. Upon bacterial infection, IL-22 knock-out mice displayed impaired gut epithelial regeneration, high bacterial load and increased mortality. Kumar et al., supra. Similarly, infection of IL-22 knock-out mice with influenza virus resulted in severe weight loss and impaired regeneration of tracheal and bronchial epithelial cells. Thus, IL-22 plays a pro-inflammatory role in suppressing microbial infection as well as an antiinflammatory protective role in epithelial regeneration in inflammatory responses. Much of IL-22's biological action promoting pathological inflammation and tissue repair remains to be determined. The seemingly conflicting reports on the effects of IL-22 on epithelial cells are not yet thoroughly understood. Kumar et al, supra.

Increased expression of IL-22 is detected in inflammatory bowel disorder (IBD) patients. See e.g., Wolk et ah, 2007, J. Immunology, 178:5973; Andoh et ah, 2005, Gastroenterology, 129:969. IBDs such as Crohn's disease (CD) and ulcerative colitis (UC) are thought to result from a dysregulated immune response to the commensal microflora present in the gut. Cox et ah, 2012, Mucosal Immunol. 5:99-109. Both UC and CD are complex diseases that occur in genetically susceptible individuals who are exposed to as yet poorlydefined environmental stimuli. CD and UC are mediated by both common and distinct mechanisms and exhibit distinct clinical features. See Sugimoto et ah 2008, J. Clinical Investigation, 1 18:534-544. Mucosal healing, which is a currently targeted method of treatment for IBD, can be induced by IL-22. IL-22 triggers the activation of STAT3, serum amyloid a, and RegIIIbeta.

In UC, inflammation occurs primarily in the mucosa of the colon and the rectum, leading to debilitating conditions including diarrhea, rectal bleeding, and weight loss. It is thought that UC is largely caused by an inappropriate inflammatory response by the host to intestinal microbes penetrating through a damaged epithelial barrier (Xavier and Podolsky, 2007, Nature 448:427-434). Crohn's disease is characterized by intestinal infilatratoin of activated immune cells and distortion of the intestinal architechture. See Wolk et ah, supra.

In another aspect, the invention provides methods of treating any one or combination of the following diseases using an IL-22 polypeptide or an IL-22 Fc fusion protein of this invention: Type II diabetes, Type II diabetes with morbid obesity, wounds (including diabetic wounds and diabetic ulcers), burns, ulcers (including pressure ulcer and venous ulcer), graft versus host disease (GVHD), microbial infection, acute kidney injury, acute pancreatitis, wounds, cardiovascular conditions, metabolic syndrome, acute endotoxemia, sepsis, atherosclerosis, cardiovascular disease, metabolic syndrome, endotoxemia (acute and mild), sepsis, acute coronary heart disease, hypertension, dyslipemia, obesity, hyperglycemia, lipid metabolism disorders, hepatitis, acute hepatitis, renal failure, acute renal failure, acute kidney injury, renal draft failure, post cadaveric renal transplant delayed graft function, contrast induced nephropathy, pancreatitis, acute pancreatitis, liver fibrosis and lung fibrosis. In certain embodiments, acute pancreatitis can be mild to moderate to severe disease. In certain embodiments, acute pancreatitis includes disease post ERCP (endoscopic retrograde cholangiopancreatography). In some further embodiments, the patient to be treated for the above disease is in need of a change in his HDL/LDL lipid profile, which IL-22 polypeptide or IL-22 Fc fusion proteins can alter in the patient to increase HDL and decrease LDL. In a related aspect, the invention provides uses of an IL-22 polypeptide or an IL-22 Fc fusion protein in the preparation of a medicament for the treatment of any one or combinations of the above diseases.

In one aspect, the invention features an IL-22 Fc fusion protein comprising an IL-22 polypeptide linked to an Fc region. In some embodiments, the IL-22 polypeptide is linked to an Fc region by a linker. In some embodiments, the IL-22 polypeptide is glycosylated, and wherein the IL-22 Fc fusion protein has a sialic acid content in the range of from 8 to 12 moles of sialic acid per mole of the IL- 22 Fc fusion protein. In certain aspects, 8 to 12 moles of sialic acid per mole of the IL-22 Fc fusion protein means that 8 to 12 sialic acid moieties are comprised in one mole of the IL-22 fusion protein. In some embodiments, the IL-22 Fc fusion protein has a sialic acid content in the range of 8 to 9 moles of sialic acid per mole of the IL-22 Fc fusion protein.

The term "Fc polypeptide" or "Fc region" as used herein includes native and mutein forms of polypeptides derived from the Fc region of an antibody. Truncated forms of such polypeptides containing the hinge region that promotes dimerization also are included. In certain embodiments, the Fc region comprises an antibody CH2 and CH3 domain. Along with extended serum half-life, fusion proteins comprising Fc moieties (and oligomers formed therefrom) offer the advantage of facile purification by affinity chromatography over Protein A or Protein G columns. Preferred Fc regions are derived from human IgG, which includes IgG1, IgG2, IgG3, and IgG4. Herein, specific residues within the Fc are identified by position. All Fc positions are based on the EU numbering scheme.

One of the functions of the Fc portion of an antibody is to communicate to the immune system when the antibody binds its target. This is considered "effector function." Communication leads to antibody-dependent cellular cytotoxicity (ADCC), antibody-dependent cellular phagocytosis (ADCP), and/or complement dependent cytotoxicity (CDC). ADCC and ADCP are mediated through the binding of the Fc to Fc receptors on the surface of cells of the immune system. CDC is mediated through the binding of the Fc with proteins of the complement system, e.g., C1q.

The IgG subclasses vary in their ability to mediate effector functions. For example, IgG1 is much superior to IgG2 and IgG4 at mediating ADCC and CDC. Thus, in embodiments wherein effector function is undesirable, an IgG2 Fc would be preferred. IgG2 Fc-containing molecules, however, are known to be more difficult to manufacture and have less attractive biophysical properties, such as a shorter half-life, as compared to IgG1 Fc-containing molecules.

The effector function of an antibody can be increased, or decreased, by introducing one or more mutations into the Fc. Embodiments of the invention include IL-22 mutein Fc fusion proteins having an Fc engineered to increase effector function (U.S. 7,317,091 and Strohl, Curr. Opin. Biotech., 20:685-691, 2009. Exemplary IgG1 Fc molecules having increased effector function include those having the following substitutions: S239D/I332E; S239D/A330S/I332E; S239D/A330L/I332E; S298A/D333A/K334A; P247I/A339D; P247I/A339Q; D280H/K290S; D280H/K290S/S298D; D280H/K290S/S298V; F243L/R292P/Y300L; F243L/R292P/Y300L/P396L; F243L/R292P/Y300L/V305I/P396L; G236A/S239D/I332E; K326A/E333A; K326W/E333S; K290E/S298G/T299A; K290N/S298G/T299A; K290E/S298G/T299A/K326E; or K290N/S298G/T299A/K326E.

Another method of increasing effector function of IgG Fc-containing proteins is by reducing the fucosylation of the Fc. Removal of the core fucose from the biantennary complex-type oligosachharides attached to the Fc greatly increased ADCC effector function without altering antigen binding or CDC effector function. Several ways are known for reducing or abolishing fucosylation of Fc-containing molecules, e.g., antibodies. These include recombinant expression in certain mammalian cell lines including a FUT8 knockout cell line, variant CHO line Lec13, rat hybridoma cell line YB2/0, a cell line comprising a small interfering RNA specifically against the FUT8 gene, and a cell line coexpressing β-1,4-N-acetylglucosaminyltransferase III and Golgi α-mannosidase II. Alternatively, the Fc-containing molecule may be expressed in a non-mammalian cell such as a plant cell, yeast, or prokaryotic cell, e.g., E. coli.

In some embodiments of the invention, IL-22-Fc fusion proteins comprise an Fc engineered to decrease effector function. Exemplary Fc molecules having decreased effector function include those having the following substitutions: N297A or N297Q (IgG1); L234A/L235A (IgG1); V234A/G237A (IgG2); L235A/G237A/E318A (IgG4); H268Q/V309L/A330S/A331S (IgG2); C220S/C226S/C229S/P238S (IgG1); C226S/C229S/E233P/L234V/L235A (IgG1); L234F/L235E/P331S (IgG1); or S267E/L328F (IgG1).

It is known that human IgG1 has a glycosylation site at N297 (EU numbering system) and glycosylation contributes to the effector function of IgG1 antibodies. An exemplary IgG1 sequence is provided in SEQ ID NO:5. Groups have mutated N297 in an effort to make aglycosylated antibodies. The mutations have focuses on substituting N297 with amino acids that resemble asparagine in physiochemical nature such as glutamine (N297Q) or with alanine (N297A) which mimics asparagines without polar groups.

As used herein, "aglycosylated antibody" or "aglycosylated fc" refers to the glycosylation status of the residue at position 297 of the Fc. An antibody or other molecule may contain glycosylation at one or more other locations but may still be considered an aglycosylated antibody or aglcosylated Fc-fusion protein.

In the effort to make an effector functionless IgG1 Fc, it was discovered that mutation of amino acid N297 of human IgG1 to glycine, i.e., N297G, provides far superior purification efficiency and biophysical properties over other amino acid substitutions at that residue. Thus, in some embodiments, the IL-22-Fc fusion protein comprises a human IgG1 Fc having a N297G substitution.

An Fc comprising a human IgG1 Fc having the N297G mutation may also comprise further insertions, deletions, and substitutions. In certain embodiments the human IgG1 Fc comprises the N297G substitution and is at least 90% identical, at least 91% identical, at least 92% identical, at least 93% identical, at least 94% identical, at least 95% identical, at least 96% identical, at least 97% identical, at least 98% identical, or at least 99% identical to the amino acid sequence set forth in SEQ ID NO:5. In a particularly preferred embodiment, the C-terminal lysine residue is substituted or deleted. The amino acid sequence of human IgG1 comprising the N297G substitution and deletion of the C-terminal lysine is set forth in SEQ ID NO:6.

DKTHTCPPCPAPELLGGPSVFLFPPKPKDTLMISRTPEVTCVVVDVSHE
DPEVKFNWYVDGVEVHNAKTKPREEQYNSTYRVVSVLTVLHQDWLNGKEYKCKV
SNKALPAPIEKTISKAKGQPREPQVYTLPPSREEMTKNQVSLTCLVKGFYPSDIAVEW
ESNGQPENNYKTTPPVLDSDGSFFLYSKLTVDKSRWQQGNVFSCSVMHEALHNHYT
QKSLSLSPGK (SEQ ID NO:5)

DKTHTCPPCPAPELLGGPSVFLFPPKPKDTLMISRTPEVTCVVVDVSHE
DPEVKFNWYVDGVEVHNAKTKPREEQYGSTYRVVSVLTVLHQDWLNGKEYKCKV
SNKALPAPIEKTISKAKGQPREPQVYTLPPSREEMTKNQVSLTCLVKGFYPSDIAVEW
ESNGQPENNYKTTPPVLDSDGSFFLYSKLTVDKSRWQQGNVFSCSVMHEALHNHYT
QKSLSLSPG (SEQ ID NO:6)

A glycosylated IgG1 Fc-containing molecules were shown to be less stable than glycosylated IgG1 Fc-containing molecules. The Fc region may be further engineered to increase the stability of the aglycosylated molecule. In some embodiments, one or more amino acids are substituted to cysteine so to form di-sulfide bonds in the dimeric state. Residues V259, A287, R292, V302, L306, V323, or I332 of the amino acid sequence set forth in SEQ ID NO:3 may be substituted with cysteine. In preferred embodiments, specific pairs of residues are substitution such that they preferentially form a di-sulfide bond with each other, thus limiting or preventing di-sulfide bond scrambling. Preferred pairs include, but are not limited to, A287C and L306C, V259C and L306C, R292C and V302C, and V323C and I332C.

Additional mutations that may be made to the IgG1 Fc include those facilitate heterodimer formation amongst Fc-containing polypeptides. In some embodiments, Fc region is engineering to create "knobs" and "holes" which facilitate heterodimer formation of two different Fc-containing polypeptide chains when co-expressed in a cell. U.S. 7,695,963. In other embodiments, the Fc region is altered to use electrostatic steering to encourage heterodimer formation while discouraging homodimer formation of two different Fc-containing polypeptide when co-expressed in a cell. WO 09/089,004. Preferred heterodimeric Fc include those wherein one chain of the Fc comprises D399K and E356K substitutions and the other chain of the Fc comprises K409D and K392D substitutions. In other embodiments, one chain of the Fc comprises D399K, E356K, and E357K substitutions and the other chain of the Fc comprises K409D, K392D, and K370D substitutions.

Also disclosed herein are uses of an IL-22 Fc fusion protein described herein in the preparation of a medicament for the treatment of IBD, including UC and CD, in a subject in need thereof. In a related disclosure are uses of an IL-22 Fc fusion protein described herein in the preparation of a medicament for inhibiting microbial infection in the intestine, or preserving goblet cells in the intestine during a microbial infection in a subject in need thereof. In yet another disclosure are uses of an IL-22 Fc fusion protein described herein in the preparation of a medicament for enhancing epithelial cell integrity, epithelial cell proliferation, epithelial cell differentiation, epithelial cell migration or epithelial wound healing in the intestine, in a subject in need thereof. In a related disclosure areuses of an IL-22 polypeptide or IL-22 Fc fusion protein in the preparation of a medicament for treating a cardiovascular condition, metabolic syndrome, atherosclerosis, acute kidney injury, acute pancreatitis, accelerating, promoting or improving wound healing, including without limitation, healing of a chronic wound, diabetic wound, infected wound, pressure ulcer or diabetic foot ulcer, in a subject in need thereof.

The IL-22-based compositions (which in certain embodiments, include IL-22 Fc fusion proteins, and IL-22 polypeptide or agonists) herein will be formulated, dosed, and administered in a fashion consistent with good medical practice. Factors for consideration in this context include the particular disorder being treated, the particular mammal being treated, the clinical condition of the individual subject, the cause of the disorder, the site of delivery of the agent, the method of administration, the scheduling of administration, and other factors known to medical practitioners. In one embodiment, the composition can be used for increasing the duration of survival of a human subject susceptible to or diagnosed with the disease or condition disease. Duration of survival is defined as the time from first administration of the drug to death.

Pharmaceutical formulations are prepared using standard methods known in the art by mixing the active ingredient having the desired degree of purity with one or more optional pharmaceutically acceptable carriers (Remington 's Pharmaceutical Sciences 16th edition, Osol, A. Ed. (1980) and Remington's Pharmaceutical Sciences 20th edition, ed. A. FGennaro, 2000, Lippincott, Williams & Wilkins, Philadelphia, Pa), in the form of lyophilized formulations or aqueous solutions. Pharmaceutically acceptable carriers are generally nontoxic to recipients at the dosages and concentrations employed, and include, but are not limited to: buffers such as phosphate, citrate, and other organic acids; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m- cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as polyethylene glycol (PEG). Exemplary pharmaceutically acceptable carriers herein further include insterstitial drug dispersion agents such as soluble neutral-active hyaluronidase glycoproteins (sHASEGP), for example, human soluble PH-20 hyaluronidase glycoproteins, such as rHuPH20 (HYLENEX^{®}, Baxter International, Inc.). Certain exemplary sHASEGPs and methods of use, including rHuPH20, are described in US Patent Publication Nos. 2005/0260186 and 2006/0104968. In one aspect, a sHASEGP is combined with one or more additional glycosaminoglycanases such as chondroitinases.

Optionally, but preferably, the formulation contains a pharmaceutically acceptable salt, preferably sodium chloride, and preferably at about physiological concentrations. Optionally, the formulations of the invention can contain a pharmaceutically acceptable preservative. In some embodiments the preservative concentration ranges from 0.1 to 2.0%, typically v/v. Suitable preservatives include those known in the pharmaceutical arts. Benzyl alcohol, phenol, m-cresol, methylparaben, benzalkonium chloride and propylparaben are preferred preservatives. Optionally, the formulations of the invention can include a pharmaceutically acceptable surfactant at a concentration of 0.005 to 0.02%.

In some embodiments are dosages for the IL-22-based therapeutics. For example, depending on the type and severity of the disease, about 1 µg/kg to 15 mg/kg (e.g. 0.1-20 mg/kg) of polypeptide is an initial candidate dosage for administration to the subject, whether, for example, by one or more separate administrations, or by continuous infusion. A typical daily dosage might range from about Iµ-g/kg to 100 mg/kg or more, depending on the factors mentioned above. For repeated administrations over several days or longer, depending on the condition, the treatment is sustained until a desired suppression of disease symptoms occurs. However, other dosage regimens can be useful. The progress of this therapy is easily monitored by conventional techniques and assays.

For the prevention or treatment of disease, the appropriate dosage of a polypeptide of the invention (when used alone or in combination with one or more other additional therapeutic agents) will depend on the type of disease to be treated, the type of polypeptide, the severity and course of the disease, whether the polypeptide is administered for preventive or therapeutic purposes, previous therapy, the subject's clinical history and response to the polypeptide, and the discretion of the attending physician. The polypeptide is suitably administered to the subject at one time or over a series of treatments. Depending on the type and severity of the disease, about 1 µg/kg to 20 mg/kg (e.g. 0.1 mg/kg- 15mg/kg) of the polypeptide can be an initial candidate dosage for administration to the subject, whether, for example, by one or more separate administrations, or by continuous infusion. One typical daily dosage might range from about 1 µg/kg to 100 mg/kg or more, depending on the factors mentioned above. For repeated administrations over several days or longer, depending on the condition, the treatment would generally be sustained until a desired suppression of disease symptoms occurs. One exemplary dosage of the polypeptide would be in the range from about 0.05 mg/kg to about 20 mg/kg. Thus, one or more doses of about 0.5 mg/kg, 2.0 mg/kg, 4.0 mg/kg, 10 mg/kg, 12 mg/kg, 15 mg/kg, or 20 mg/kg (or any combination thereof) may be administered to the subject. In certain embodiments, about 0.5 mg/kg, 1.0 mg.kg, 2.0 mg/kg, 3.0 mg/kg, 4.0 mg/kg, 5.0 mg/kg, 6.0 mg/kg, 7.0 mg/kg, 8.0 mg/kg, 9.0 mg/kg, 10 mg/kg, 12 mg/kg, 15 mg/kg, or 20 mg/kg (or any combination thereof) may be administered to the subject. Such doses may be administered intermittently, e.g. every week, every two weeks, or every three weeks (e.g. such that the subject receives from about two to about twenty, or e.g. about six doses of the polypeptide). An initial higher loading dose, followed by one or more lower doses may be administered. An exemplary dosing regimen comprises administering an initial loading dose of about 4 mg/kg, followed by a weekly maintenance dose of about 2 mg/kg of the antibody. However, other dosage regimens may be useful. The progress of this therapy is easily monitored by conventional techniques and assays.

An IL-22 Fc fusion protein for use as a medicament is disclosed herein. In further aspects, an IL-22 Fc fusion protein for use in treating IBD, including UC and CD, is provided. In certain embodiments, an IL-22 Fc fusion protein for use in a method of treatment is provided. In certain embodiments, the invention provides an IL-22 Fc fusion protein for use in a method of treating an individual having UC or CD comprising administering to the individual an effective amount of the IL-22 Fc fusion protein. In one such embodiment, the method further comprises administering to the individual an effective amount of at least one additional therapeutic agent, e.g., as described below. In further embodiments, the invention provides an IL-22 Fc fusion protein for use in enhancing epithelial proliferation, differentiation and/or migration. In certain particular embodiments, the epithelial tissue is intestinal epithelial tissue. In certain embodiments, the invention provides an IL-22 Fc fusion protein for use in a method of enhancing epithelial proliferation, differentiation and/or migration in an individual comprising administering to the individual an effective amount of the IL-22 Fc fusion protein to enhance epithelial proliferation, differentiation and/or migration. In yet other embodiments, the invention provides an IL-22 Fc fusion protein for use in treating diabetes, especially type II diabetes, diabetic wound healing, metabolic syndromes and atherosclerosis. In certain embodiments, the invention provides an IL-22 Fc fusion protein for use in a method of treating diabetes, especially type II diabetes, diabetic wound healing, metabolic syndromes and atherosclerosis in an individual comprising administering to the individual an effective amount of the IL-22 Fc fusion protein. See application serial number 61/800795, entitled "Using an IL-22 polypeptide for wound healing," and application serial number 61/801144, entitled "Methods of treating cardiovascular conditions and metabolic syndrome using an IL-22 polypeptide," both filed on March 15, 2013. An "individual" or "subject" or "patient" according to any of the above embodiments is preferably a human.

Also disclosed herein is the use of an IL-22 polypeptide or IL-22 Fc fusion protein in the manufacture or preparation of a medicament. In one embodiment, the medicament is for treatment of IBD and wound healing. In a further embodiment, the medicament is for use in a method of treating IBD and wound healing comprising administering to an individual having IBD an effective amount of the medicament. In one such embodiment, the method further comprises administering to the individual an effective amount of at least one additional therapeutic agent, e.g., as described below. In a further embodiment, the medicament is for suppressing inflammatory response in the gut epithelial cells. In a further embodiment, the medicament is for use in a method of enhancing epithelial proliferation, differentiation and/or migration in an individual comprising administering to the individual an amount effective of the medicament to enhance epithelial proliferation, differentiation and/or migration. An "individual" according to any of the above embodiments may be a human.

In a further aspect, the invention provides a method for treating IBD, including UC and CD. In one embodiment, the method comprises administering to an individual having IBD an effective amount of an IL-22 polypeptide or an IL-22 Fc fusion protein. In one such embodiment, the method further comprises administering to the individual an effective amount of at least one additional therapeutic agent, as described below. An "individual" according to any of the above embodiments may be a human.

As provided herein, a protease inhibitor protects the protein of the present invention from cleavage. In another embodiment, the present invention provides that a protease inhibitor protects insulin of the present invention from cleavage. In another, embodiment, the present invention provides that a protease inhibitor facilitates the protein absorption in the intestine of a subject. In another, embodiment, the present invention provides that a protease inhibitor facilitates the absorption of insulin in the intestine of a subject.

In another, embodiment, a serpin is: Alpha 1-antitrypsin, Antitrypsin-related protein, Alpha 1 anti chymotrypsin, Kallistatin, Protein C inhibitor, Cortisol binding globulin, Thyroxine-binding globulin, Angiotensinogen, Centerin, Protein Z-related protease inhibitor, Vaspin, Monocyte neutrophil elastase inhibitor, Plasminogen activator inhibitor-2, Squamous cell carcinoma antigen-1 (SCCA-1), Squamous cell carcinoma antigen-2 (SCCA-2), Maspin, PI-6, Megsin, PI-8, PI-9, Bomapin, Yukopin, Hurpin/Headpin, Antithrombin, Heparin cofactor II, Plasminogen activator inhibitor 1, Glia derived nexin / Protease nexin I, Pigment epithelium derived factor, Alpha 2-antiplasmin, Complement 1- inhibitor, 47 kDa Heat shock protein (HSP47), Neuroserpin, or Pancpin.

In another embodiment, the present invention provides that the protease inhibitor is a trypsin inhibitor such as but not limited to: Lima bean trypsin inhibitor, Aprotinin, soy bean trypsin inhibitor (SBTI), or Ovomucoid. In another embodiment, the present invention provides that the protease inhibitor is a Cysteine protease inhibitor. In another, embodiment, the present invention provides that Cysteine protease inhibitors of the invention comprise: cystatin,type 1 cystatins (or stefins), Cystatins of type 2,human cystatins C, D, S, SN, and SA, cystatin E/M, cystatin F, type 3 cystatins, or kininogens. In another embodiment, the present invention provides that the protease inhibitor is a Threonine protease inhibitor. In another, embodiment, the present invention provides that Threonine protease inhibitors of the invention comprise: Bortezomib, MLN-519, ER-807446, TMC-95A. In another embodiment, the present invention provides that the protease inhibitor is an Aspartic protease inhibitor. In another, embodiment, the present invention provides that Aspartic protease inhibitors of the invention comprise: a2-Macroglobulin, Pepstatin A, Aspartic protease inhibitor 11, Aspartic protease inhibitor 1, Aspartic protease inhibitor 2, Aspartic protease inhibitor 3, Aspartic 20 protease inhibitor 4, Aspartic protease inhibitor 5, Aspartic protease inhibitor 6, Aspartic protease inhibitor 7, Aspartic protease inhibitor 8, Aspartic protease inhibitor 9, Pepsin inhibitor Dit33, Aspartyl protease inhibitor, or Protease A inhibitor 3. In another embodiment, the present invention provides that the protease inhibitor is a Metalloprotease inhibitor. In another, embodiment, the present invention provides that Metalloprotease inhibitors of the invention comprise: Angiotensin-1-converting enzyme inhibitory peptide,

Antihemorragic factor BJ46a, Beta-casein, Proteihase inhibitor CeKI, Venom metalloproteinase inhibitor DM43, Carboxypeptidase A inhibitor, smpl, IMPI, Alkaline proteinase, inh, Latexin, Carboxypeptidase inhibitor, Antihemorragic factor HSF, Testican-3, SPOCK3, TIMP1, Metalloproteinase inhibitor 1, Metalloproteinase inhibitor 2, TEMP2, Metalloproteinase inhibitor 3, TIMP3, Metalloproteinase inhibitor 4, TIMP4, Putative metalloproteinase inhibitor tag-225, Tissue inhibitor of metalloprotease, WAP, kazal, immunoglobulin, or kunitz and NTR domain-containing protein 1.

In some embodiments, a protease inhibitor is a suicide inhibitor, a transition state inhibitor, or a chelating agent. In some embodiments, the protease inhibitor of the present invention is: AEBSFHCl,(epsilon)-aminocaproic acid,(alpha) 1-antichymotypsin, antipain, antithrombin III, (alpha) 1 antitrypsin ([alpha] 1-proteinase inhibitor),APMSF-HC1 (4-amidinophenyl-methane sulfonylfluoride),sprotinin, benzamidine-HCl,chymostatin, DFP (diisopropylfluoro-phosphate), leupeptin, PEFABLOC^{®} SC (4-(2-Aminoethyl)-benzenesulfonyl fluoride hydrochloride), PMSF (phenylmethyl sulfonyl fluoride), TLCK (1-Chloro-3-tosylamido-7-amino-2-heptanone HCl), TPCK (1-Chloro-3tosylamido-4-phenyl-2-butanone), pentamidine isethionate, pepstatin, guanidium, alpha2macroglobulin,a chelating agent of zinc, or iodoacetate, zinc. Each possibility represents a separate embodiment of the present invention.

In another embodiment, the amount of a protease inhibitor utilized in methods and compositions of the present invention is 0.1 mg/dosage unit. In another embodiment, the amount of a protease inhibitor is 0.2 mg/dosage unit. In another embodiment, the amount is 0.3 mg/dosage unit. In another embodiment, the amount is 0.4 mg/dosage unit. In another embodiment, the amount is 0.6 mg/dosage unit. In another embodiment, the amount is 0.8 mg/dosage unit. In another embodiment, the amount is 1 mg/dosage unit. In another embodiment, the amount is 1.5 mg/dosage unit. In another embodiment, the amount is 2 mg/dosage unit. In another embodiment, the amount is 2.5 mg/dosage unit. In another embodiment, the amount is 3 mg/dosage unit. In another embodiment, the amount is 5 mg/dosage unit. In another embodiment, the amount is 7 mg/dosage unit. In another embodiment, the amount is 10 mg/dosage unit. In another embodiment, the amount is 12 mg/dosage unit. In another embodiment, the amount is 15 mg/dosage unit. In another embodiment, the amount is 20 mg/dosage unit. In another embodiment, the amount is 30 mg/dosage unit. In another embodiment, the amount is 50 mg/dosage unit. In another embodiment, the amount is 70 mg/dosage unit. In another embodiment, the amount is 100 mg/dosage unit.

In another embodiment, compositions of the present invention comprise a substance that enhances absorption of a protein of the invention through an intestinal mucosal barrier. In another embodiment, compositions of the present invention further comprise a substance that enhances absorption of IL-22 through an intestinal mucosal barrier. In another embodiment, compositions of the present invention further comprise a substance that reduces the degradation of IL-22 in the digestive system. In another embodiment, compositions of the present invention further comprise a substance that reduces the degradation of IL-22 in the stomach. In another embodiment, compositions of the present invention further comprise a substance that reduces the degradation of IL-22 in the intestine. Such a substance is referred to herein as an "enhancer." As provided herein, enhancers, when used together with omega-3 fatty acids or a protease inhibitor, enhance the ability of a protein, such as IL-22, to be absorbed in the intestine. As provided herein, enhancers, when used together with omega-3 fatty acids and a protease inhibitor, enhance the ability of insulin to be absorbed in the intestine. As provided herein, enhancers, when used together with omega-3 fatty acids and a protease inhibitor, enhance the ability of IL-22 to be absorbed in the intestine.

The invention provides an absorption enhancer, wherein the absorption enhancer is N(8-[2-hydroxybenzoyl]amino)caprylic acid (NAC) or a salt of said NAC. Other enhancers may be didecanoylphosphatidylcholine (DDPC). Other enhancers may be a chelating agent such as ethylenediaminetetraacetic acid (EDTA) or egtazic acid EGTA. EDTA may be sodium-EDTA. Other enhancers may be NO donor. Other enhancers may be a bile acid, glycineconjugated form of a bile acid, or an alkali metal salt. Absorption enhancement may be achieved through utilization of a combination of a-galactosidase and p-mannanase. Other enhancers may be a fatty acid such as sodium caprate. Other enhancer may be sodium glycocholate. Other enhancers may be sodium salicylate. Other enhancers may be n-dodecyl-p-Dmaltopyranoside. Surfactants may serve as absorption enhancers. Other enhancers may be chitisan such as N, N, N -trimethyl chitosan chloride (TMC).

NO donors of the present disclosure comprise 3-(2-Hydroxy-1-(1methylethyl)-2-nitrosohydrazino)-1-propanamine, N-ethyl-2-(1-ethyl-hydroxy-2-nitrosohydrazino) ethanamine, or S-Nitroso-N-acetylpenicillamine.

The bile acid may be cholic acid. The bile acid may be chenodeoxycholic acid. The bile acid may be taurocholic acid. The bile acid may be taurochenodeoxycholic acid. The bile acid may be glycocholic acid. The bile acid may be glycochenocholic acid. The bile acid may be 3 beta-monohydroxychloric acid. IThe bile acid may be lithocholic acid. The bile acid may be 5 beta-cholanic acid. The bile acid may be 3,12-diol-7-one-5 beta-cholanic acid. the bile acid may be 3 alphahydroxy-1 2-ketocholic acid. The bile acid may be 3 beta-hydroxy-12-ketocholic acid. The bile acid may be 12 alpha-3 beta-dihydrocholic acid. The bile acid may be ursodesoxycholic acid.

The enhancer may be a nonionic surfactant. The enhancer may be a nonionic polyoxyethylene ether surface active agent (e.g one having an HLB value of 6 to 19, wherein the average number of polyoxyethylene units is 4 to 30). The enhancer may be an anionic surface active agents. The enhancer may be a cationic surface active agent. The enhancer may be an ampholytic surface active agent. Zwitteruionic surfactants such as acylcarnitines may serve as absorption enhancers.

In another embodiment, the absorption enhancer is an effective oral, intra-rectal, or other gut-related absorption enhancer for macromolecular drugs. In another embodiment, the absorption enhancer is very water soluble. In another embodiment, the absorption enhancer is fully, i. e. greater than 85%, absorbed by the gastro-intestinal tract. In another embodiment, the absorption enhancer is a coarse form. In another embodiment, the absorption enhancer is micronized. In another embodiment, the absorption enhancer is amorphous. The invention provides an absorption enhancer, wherein the absorption enhancer is N(8-[2-hydroxybenzoyl]amino)caprylic acid (NAC) or a salt of said NAC. Other absorption enhancers may be N- (5-chlorosalicytoyl)-8-aminocaprylic acid (CNAC). Other absorption enhancers may be N- (1 0-[2-hydroxybenzoyl] amino) decanoic acid (SNAD). In embodiments, the absorption enhancer is N- (8- [2-hydroxybenzoyl] amino) caprylic acid (SNAC). Without being bound by theory, CNAC, SNAD, and/or SNAC can non-covalently complex with IL-22 to increase lipophilicity and/or reduce transepithelial electrical resistance. In another embodiment, the absorption enhancer is SNAC, a monosodium and/or disodium salts thereof, and any combinations thereof. Other absorption enhancers may be 8-(N-2-hydroxy-4-methoxybenzoyl)-aminocaprylic acid (4-MOAC) and pharmaceutically acceptable salts thereof and/or amorphous and polymorphic forms of 4-MOAC. Other absorption enhancers may be N-(8-[2-hydroxy-5-chlorobenzoyl]-amino)octanoic acid (also known as 8-(N-2-hydroxy-5-chlorobenzoyl)aminocaprylic acid)) (5-CNAC) and pharmaceutically acceptable salts thereof and/or amorphous and polymorphic forms of 5-CNAC. Other absorption enhancers may be 4-[(2-hydroxy-4- chlorobenzoyl)amino]butanoate (also known as 4-[(4-chloro-2-hydroxy- benzoyl)amino]butanoic acid) (4-CNAB) and pharmaceutically acceptable salts thereof, including its monosodium salt and/or amorphous and polymorphic forms of 4-CNAB .

In another embodiment, the pharmaceutical compositions of the present invention comprise a delivery effective amount of one or more of the absorption enhancers.In another embodiment, the pharmaceutical compositions of the present invention comprise an amount sufficient to deliver the active agent for the desired effect.

In another embodiment, the pharmaceutical compositions of the present invention comprise an amount of 2.5% to 99.4% by weight of an absorption enhancer. In another embodiment, the 20 pharmaceutical compositions of the present invention comprise an amount of2.5% to 10% by weight of an absorption enhancer. In another embodiment, the pharmaceutical compositions of the present invention comprise an amount of 8% to 15% by weight of a absorption enhancer. In another embodiment, the pharmaceutical compositions of the present invention comprise an amount of 10% to 20% by weight of a absorption enhancer. In another embodiment, the pharmaceutical compositions of 25 the present invention comprise an amount of 15% to 30% by weight of an absorption enhancer. In another embodiment, the pharmaceutical compositions of the present invention comprise an amount of 20% to 40% by weight of an absorption enhancer. In another embodiment, the pharmaceutical compositions of the present invention comprise an amount of 30% to 50% by weight of an absorption enhancer. In another embodiment, the pharmaceutical compositions of the present invention comprise an amount of 40% to 60% by weight of an absorption enhancer. In another embodiment, the pharmaceutical compositions of the present invention comprise an amount of 50% to 70% by weight of an absorption enhancer. In another embodiment, the pharmaceutical compositions of the present invention comprise an amount of 70% to 99.4% by weight of an absorption enhancer. In another 5 embodiment, the amount of an absorption enhancer in the present composition is a delivery effective amount and can be determined for any particular carrier or biologically or chemically active agent by methods known to those skilled in the art.

In another embodiment, the amount of enhancer utilized in methods and compositions of the present invention is 0.1 mg/dosage unit. In another embodiment, the amount of enhancer is 0.2 mg/dosage unit. In another embodiment, the amount is 0.3 mg/dosage unit. In another embodiment, the amount is 0.4 mg/dosage unit. In another embodiment, the amount is 0.6 mg/dosage unit. In another embodiment, the amount is 0.8 mg/dosage unit. In another embodiment, the amount is 1 mg/dosage unit. In another embodiment, the amount is 1.5 mg/dosage unit. In another embodiment, the amount is 2 mg/dosage unit. In another embodiment, the amount is 2.5 mg/dosage unit. In another embodiment, the amount is 3 mg/dosage unit. In another embodiment, the amount is 5 mg/dosage unit. In another embodiment, the amount is 7 mg/dosage unit. In another embodiment, the amount is 10 mg/dosage unit. In another embodiment, the amount is 12 mg/dosage unit. In another embodiment, the amount is 15 mg/dosage unit. In another embodiment, the amount is 20 mg/dosage unit. In another embodiment, the amount is 30 mg/dosage unit. In another embodiment, the amount is 50 mg/dosage unit. In another embodiment, the amount is 70 mg/dosage unit. In another embodiment, the amount is 100 mg/dosage unit.

In another embodiment, compositions of the present invention further comprise a coating that inhibits digestion of the composition in a subject's stomach. In one embodiment, coating inhibits digestion of the composition in a subject's stomach. In one embodiment, the coated dosage forms of the present invention release drug when pH move towards alkaline range. In one embodiment, coating is a monolayer, wherein in other embodiments coating applied in multilayers. In one embodiment, coating is a bioadhesive polymer that selectively binds the intestinal mucosa and thus enables drug release in the attachment site. In one embodiment, the enteric coating is an enteric film coating. In some embodiment, coating comprises biodegradable polysaccharide, chitosan, aquateric aqueous, aquacoat ECD, azo polymer, cellulose acetate phthalate, cellulose acetate trimelliate, hydroxypropylmethyl cellulose phthalate, gelatin, poly vinyl acetate phthalate, hydrogel, pulsincap, or a combination thereof. In one embodiment, pH sensitive coating will be used according to the desired release site and/or profile as known to one skilled in the art.

In one embodiment, the coating is an enteric coating. Methods for enteric coating are well known in the art, and are described, for example, in Siepmann F, Siepmann J et al, Blends of aqueous polymer dispersions used for pellet coating: importance of the particle size. J Control Release 2005; 105(3): 226-39; and Huyghebaert N, Vermeire A, Remon JP. In vitro evaluation of coating polymers for enteric coating and human ileal targeting. Int J Pharm 2005; 298(1): 26-37. Each method represents a separate embodiment of the present invention.

In another embodiment, Eudragit^{®}, an acrylic polymer, is used as the enteric coating. The use of acrylic polymers for the coating of pharmaceutical preparations is well known in the art. Eudragit Acrylic Polymers have been shown to be safe, and are neither absorbed nor metabolized by the body, but rather are eliminated.

In another embodiment, the coating is a gelatin coating. In another embodiment, microencapsulation is used to protect the insulin against decomposition in the stomach. In another embodiment, the coating is a gelatin coating. In another embodiment, microencapsulation is used to protect Exenatide against decomposition in the stomach. Methods for applying a gelatin coating and for microencapsulation are well known in the art. Each method represents a separate embodiment of the present invention.

In another embodiment, the coating is a film-coating. In another embodiment, the coating is ethylcellulose. In another embodiment, the coating is a water-based dispersion of ethylcellulose, e.g. hydroxypropylmethylcelullose (HPMC) E15. In another embodiment, the coating is a gastro-resistant coating, e.g. a polymer containing carboxylic acid groups as a functional moiety. In another embodiment, the coating is a monolithic matrix. In another embodiment, the coating is cellulose ether (e.g. hypromellose (HPMC). Each type of coating represents a separate embodiment of the present invention.

In another embodiment, the present invention provides that the use of at least one protease inhibitor and an absorption enhancer in a single oral, intra-rectal, or other gut-related composition dramatically, unexpectedly, increase the bioavailability of a protein of the invention. In another embodiment, the present invention provides that the use of at least one protease inhibitor and an absorption enhancer in a single oral, intra-rectal, or other gut-related composition dramatically, unexpectedly, increase the bioavailability of IL-22.

In another embodiment, the present invention provides that the use of at least one protease inhibitor and SNAC in a single oral, intra-rectal, or other gut-related composition dramatically, unexpectedly, increase the bioavailability of a protein of the invention. In another embodiment, the present invention provides that the use of at least one protease inhibitor and SNAC in a single oral, intra-rectal, or other gut-related composition dramatically, unexpectedly, increase the bioavailability of IL-22.

The present disclosure describes that the use of at least one protease inhibitor and SNAD in a single oral, intra-rectal, or other gut-related composition dramatically, unexpectedly, increase the bioavailability of a protein of the invention. The present disclosure also describes that the use of at least one protease inhibitor and SNAD in a single oral, intra-rectal, or other gut-related composition dramatically, unexpectedly, increase the bioavailability of IL-22.

In another embodiment, the present invention provides that the use of a protease inhibitor and SNACin a single oral, intra-rectal, or other gut-related composition dramatically, unexpectedly, increase the bioavailability of IL-22 in a human subject by at least 10%. In another embodiment, the present invention provides that the use of a protease inhibitor and SNACin a single oral, intra-rectal, or other gut-related composition dramatically, unexpectedly, increase the bioavailability of IL-22 in a human subject by at least 20%. In another embodiment, the present invention provides that the use of a protease inhibitor and SNACin a single oral, intra-rectal, or other gut-related composition dramatically, unexpectedly, increase the bioavailability of IL-22 in a human subject by at least 30%. In another embodiment, the present invention provides that the use of a protease inhibitor and SNAC in a single oral, intra-rectal, or other gut-related composition dramatically, unexpectedly, increase the bioavailability of IL-22 in a human subject by at least 40%. In another embodiment, the present invention provides that the use of a protease inhibitor and SNACin a single oral, intra-rectal, or other gut-related composition dramatically, unexpectedly, increase the bioavailability of IL-22 in a human subject by at least 50%. In another embodiment, the present invention provides that the use of a protease inhibitor and SNAC in a single oral, intra-rectal, or other gut-related composition dramatically, unexpectedly, increase the bioavailability of IL-22 in a human subject by at least 60%. In another embodiment, the present invention provides that the use of a protease inhibitor and SNAC in a single oral, intra-rectal, or other gut-related composition dramatically, unexpectedly, increase the bioavailability of IL-22 in a human subject by at least 70%. In another embodiment, the present invention provides that the use of a protease inhibitor and SNAC in a single oral, intra-rectal, or other gut-related composition dramatically, unexpectedly, increase the bioavailability of IL-22 in a human subject by at least 80%. In another embodiment, the present invention provides that the use of a protease inhibitor and SNAC in a single oral, intra-rectal, or other gut-related composition dramatically, unexpectedly, increase the bioavailability of IL-22 in a human subject by at least 90%. In another embodiment, the present invention provides that the use of a protease inhibitor and SNAC in a single oral, intra-rectal, or other gut-related composition dramatically, unexpectedly, increase the bioavailability of IL-22 in a human subject by at least 100%.

In another embodiment, the present invention provides a method for oral, intra-rectal, or other gut-related administration of a protein, such as IL-22, with an enzymatic activity to a subject, whereby a substantial fraction of the protein retains the enzymatic activity after absorption through an intestinal mucosal barrier of the subject, comprising administering orally, intra-rectally, or other gut-related means to the subject a pharmaceutical composition comprising the protein, a protease inhibitor, and an absorption enhancer, thereby orally, intra-rectally, or other gut-related administering a protein with an enzymatic activity to a subject.

In another embodiment, the present invention provides a method for oral, intra-rectal, or other gut-related administration of IL-22 to a subject, whereby a substantial fraction of IL-22 retains its activity after absorption through an intestinal mucosal barrier of the subject, comprising administering orally, intra-rectally, or other gut-related to the subject a pharmaceutical composition comprising IL-22 and SNAC, thereby orally, intra-rectally, or other gut-related administering IL-22 to a subject. In another embodiment, the present invention provides a method for oral, intra-rectal, or other gut-related administration of IL-22 to a subject, whereby a substantial fraction of IL-22 retains its activity after absorption through an intestinal mucosal barrier of the subject, comprising administering orally, intra-rectally, or other gut-related to the subject a pharmaceutical composition comprising IL-22, a protease inhibitor, and SNAC, thereby orally, intra-rectally, or other gut-related administering IL-22 to a subject.

In another embodiment, the present invention provides a method for oral, intra-rectal, or other gut-related administration of IL-22 to a subject, whereby a substantial fraction of the administered IL-22 retains activity after absorption through an intestinal mucosal barrier of the subject, comprising administering orally, intra-rectally, or other gut-related to the subject a pharmaceutical composition comprising IL-22, at least one protease inhibitor, SNAC, and an omega-3 fatty acid, thereby orally, intra-rectally, or other gut-related administering IL-22 to a subject. In another embodiment, the present invention provides a method for oral, intra-rectal, or other gut-related administration of IL-22 to a subject, whereby a substantial fraction of the administered IL-22 retains the enzymatic activity after absorption through an intestinal mucosal barrier of the subject, comprising administering orally, intra-rectally, or other gut-related to the subject a pharmaceutical composition comprising IL-22, at least one protease inhibitor, and SNAC, EDTA (or a salt thereof), and an omega-3 fatty acid, thereby orally, intra-rectally, or other gut-related administering IL-22 to a human subject.

In another embodiment, the present invention provides a method for treating inflammatory disorder in a human subject, comprising administering orally, intra-rectally, or other gut-related to the subject a pharmaceutical composition comprising IL-22, at least one protease inhibitor, and SNAC, thereby treating the inflammatory disorder. In another embodiment, the present invention provides a method for treating an inflammatory disorder in a human subject, comprising administering orally, intra-rectally, or other gut-related to the subject a phannaceutical composition comprising IL-22 and at least one protease inhibitor, and SNAC, and omega-3 fatty acid thereby treating the inflammatory disorder. In another embodiment, the present invention provides a method for treating an inflammatory disorder in a human subject, comprising administering orally, intra-rectally, or other gut-related to the subject a pharmaceutical composition comprising IL-22 and at least one protease inhibitor, and SNAC, EDTA (or a salt thereof), and omega-3 fatty acid thereby treating the inflammatory disorder. In some embodiments, the inflammatory disorder stated above is irritable bowel disease (IBD). In some embodiments, the inflammatory disorder is ulcerative colitis, Crohn's disease, or fistulas related to ulcerative colitis or Crohn's disease. In some embodiments, the inflammatory disorder is ulcerative colitis. In some embodiments, the inflammatory disorder is Crohn's disease. It has been shown that administration of IL-22 or IL-22-Fc into the gut allows for greater retention of IL-22 or IL-22-Fc in the gut environment, rather than systemically as compared to parenteral administration. Furthermore, oral or intra-rectal administration can prevent spike levels of IL-22 in systemic serum levels.

Also described herein is the use of a protein, such as IL-22, at least one protease inhibitor, and SNAC or SNAD in the manufacture of a medicament for oral, intra-rectal, or other gut-related administration of a protein to a subject, whereby a substantial fraction of the protein retains its activity after absorption through an intestinal mucosal barrier of the subject. The disclosure also describes the use of a protein, at least one protease inhibitor, SNAC or SNAD, and an omega-3 fatty acid in the manufacture of a medicament for oral, intra-rectal, or other gut-related administration of a protein to a subject, whereby a substantial fraction of the protein retains its activity after absorption through an intestinal mucosal barrier of the subject. The disclosure also describes the use of a protein, at least one protease inhibitor, SNAC or SNAD, Na-EDTA, and an omega-3 fatty acid in the manufacture of a medicament for oral, intra-rectal, or other gut-related administration of a protein with to a subject, whereby a substantial fraction of the protein retains its activity after absorption through an intestinal mucosal barrier of the subject. The disclosure also describes the use of a protein, such as IL-22, at least one protease inhibitor, and SNAC or SNAD in the manufacture of a medicament for oral, intra-rectal, or other gut-related administration of a protein to a subject, whereby a substantial fraction of the protein retains its activity after absorption through an intestinal mucosal barrier of the subject is for use in an inflammatory disorder. In some embodiments, the inflammatory disorder stated above is irritable bowel disease (IBD). In some embodiments, the inflammatory disorder is ulcerative colitis, Crohn's disease, or fistulas related to ulcerative colitis or Crohn's disease. In some embodiments, the inflammatory disorder is ulcerative colitis. In some embodiments, the inflammatory disorder is Crohn's disease.

In another embodiment, solid carriers/diluents for use in methods and compositions of the present invention include, but are not limited to, a gum, a starch (e.g. corn starch, pregeletanized starch), a sugar (e.g., lactose, mannitol, sucrose, dextrose), a cellulosic material (e.g. microcrystalline cellulose), an acrylate (e.g. polymethylacrylate), calcium carbonate, magnesium oxide, talc, or mixtures thereof.

In another embodiment, the compositions further comprise binders (e.g. acacia, cornstarch, gelatin, carbomer, ethyl cellulose, guar gum, hydroxypropyl cellulose, hydroxypropyl methyl cellulose, povidone), disintegrating agents (e.g. cornstarch, potato starch, alginic acid, silicon dioxide, croscarmelose sodium, crospovidone, guar gum, sodium starch glycolate), buffers (e.g., Tris-HCI., acetate, phosphate) of various pH and ionic strength, additives such as albumin or gelatin to prevent absorption to surfaces, detergents (e.g., Tween 20, Tween 80, Pluronic F68, bile acid salts), surfactants (e.g. sodium lauryl sulfate), permeation enhancers, solubilizing agents (e.g., glycerol, polyethylene glycerol), anti-oxidants (e.g., ascorbic acid, sodium metabisulfite, butylated hydroxyanisole), stabilizers (e.g. hydroxypropyl cellulose, hyroxypropylmethyl cellulose), viscosity increasing agents(e.g. carbomer, colloidal silicon dioxide, ethyl cellulose, guar gum), sweeteners (e.g. aspartame, citric acid), preservatives (e.g., Thimerosal, benzyl alcohol, parabens), lubricants (e.g. stearic acid, magnesium stearate, polyethylene glycol, sodium lauryl sulfate), flow-aids (e.g. colloidal silicon dioxide), plasticizers (e.g. diethyl phthalate, triethyl citrate), emulsifiers (e.g. carbomer, hydroxypropyl cellulose, sodium lauryl sulfate), polymer coatings (e.g., poloxamers or poloxamines), coating and film fonning agents (e.g. ethyl cellulose, acrylates, polymethacrylates) and/or adjuvants. Each of the above excipients represents a separate embodiment of the present invention.

In some embodiments, the dosage forms of the present invention are formulated to achieve an immediate release profile, an extended release profile, or a delayed release profile. In some embodiments, the release profile of the composition is determined by using specific excipients that serve for example as binders, disintegrants, fillers, or coating materials. In one embodiment, the composition will be formulated to achieve a particular release profile as known to one skilled in the art.

In one embodiment, the composition is formulated as an oral, intra-rectal, or other gut-related dosage form. In one embodiment, the composition is a solid oral, intra-rectal, or other gut-related dosage form comprising tablets, chewable tablets, or capsules. In one embodiment the capsules are soft gelatin capsules. In another embodiment, capsules as described herein are hard-shelled capsules. In another embodiment, capsules as described herein are soft-shelled capsules. In another embodiment, capsules as described herein are made from gelatine. In another embodiment, capsules as described herein are made from plant-based gelling substances like carrageenans and modified forms of starch and cellulose.

In other embodiments, controlled- or sustained-release coatings utilized in methods and compositions of the present invention include formulation in lipophilic depots (e.g. fatty acids, waxes, oils).

The preparation of phannaceutical compositions that contain an active component, for example by mixing, granulating, or tablet-forming processes, is well understood in the art. The active therapeutic ingredient is often mixed with excipients that are pharmaceutically acceptable and compatible with the active ingredient. For oral, intra-rectal, or other gut-related administration, the active ingredients of compositions of the present invention are mixed with additives customary for this purpose, such as vehicles, stabilizers, or inert diluents, and converted by customary methods into suitable forms for administration, such as tablets, coated tablets, hard or soft gelatin capsules, aqueous, alcoholic or oily solutions.

The compositions of the present invention generally may be formulated in a neutral or salt form. Pharmaceutically acceptable salts include, for example, acid addition salts (formed with free amino groups) derived from inorganic acids (e.g., hydrochloric or phosphoric acids), or from organic acids (e.g., acetic, oxalic, tartaric, mandelic, and the like). Salts formed with the free carboxyl groups can also be derived from inorganic bases (e.g., sodium, potassium, ammonium, calcium, or ferric hydroxides) or from organic bases (e.g., isopropylamine, trimethylamine, histidine, procaine and the like).

The following examples, including the experiments conducted and results achieved, are provided for illustrative purposes only and are not to be construed as limiting the present invention.

### Examples

### Example 1: Intestinal barrier crossing using a SNAC-IL-22 complex

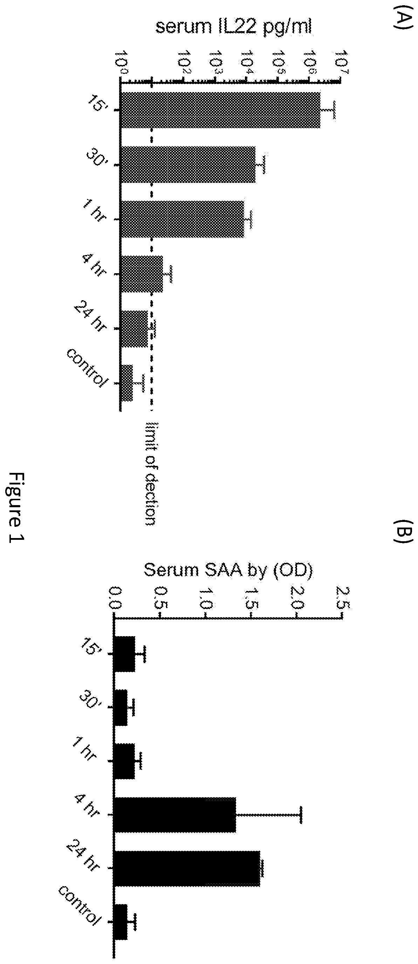

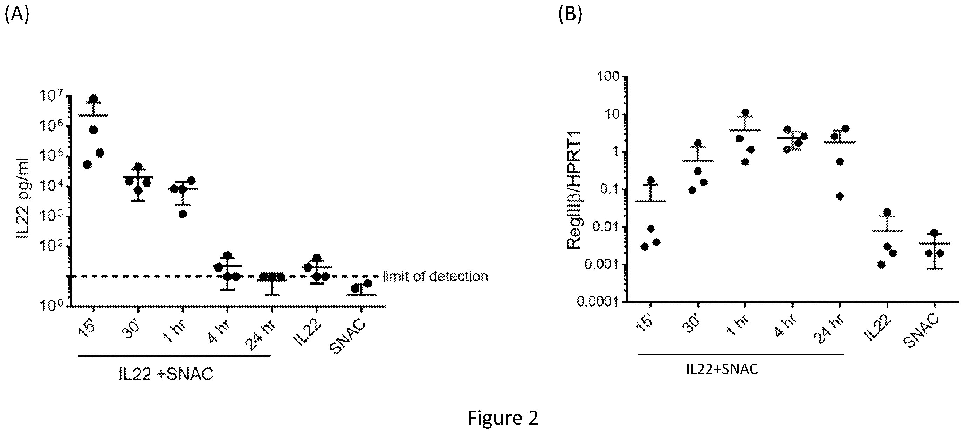

C57B1/6 mice (n=4) were intra-rectal dosed with 100 ul mixture of 1 mg/ml IL-22 and 100 mg/ml SNAC formulation. Serum were taken at indicated time for IL-22 ELISA or serum amyloid A (SAA) ELISA, respectively. Serum levels of IL-22 following intra-rectal administration of IL-22/SNAC mixture showed elevated levels of serum IL-22 which was greater than 10e6 pg/ml at 15 minutes an maintained an elevated level until at least 4 hours (Figure 1a). Serum amyloid A (SAA) is a highly conserved, acute-phase protein synthesized predominantly by the liver. During acute inflammation, serum SAA levels may rise up to 1000-fold. SAA exhibits significant immunological activity by, for example, inducing the synthesis of several cytokines and by being chemotactic for neutrophils and mast cells, and is thought to protect colon epithelium against acute injury in a mouse colitis model. Figure 1b shows elevated levels of serum SAA from liver following intra-rectal administration of IL-22/SNAC mixture, with peak levels at 24 hours post administration. Figure 2a shows the serum levels of IL-22 for each mouse tested. Figure 2b shows elevated expression of RegIIIbeta in colon following intra-rectal administration of IL-22/SNAC.

### Example 2: IL-22 Gut vs Systemic PK studies

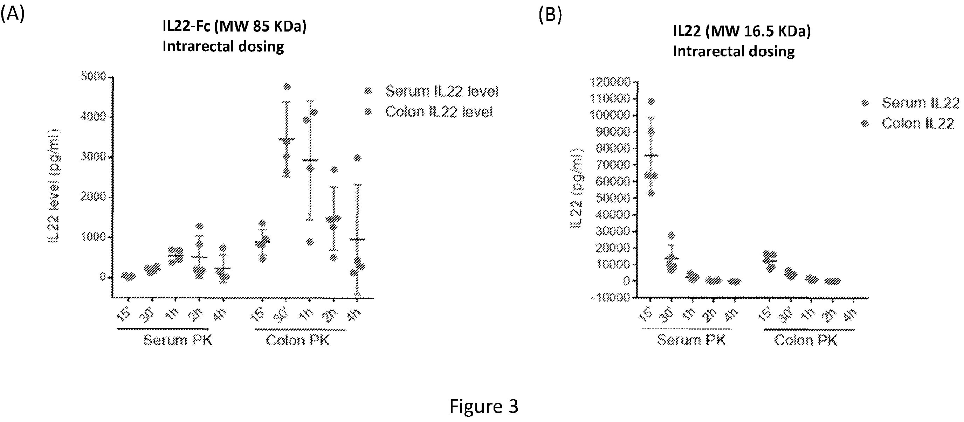

Mice were administered either IL-22 (16.5 kDa) or murine IL22Fc (85 kDa) intra-rectally to examine levels of IL-22 retained in the colon versus systemic absorption. For intra-rectal dosing, C57B1/6 mice were dosed with 100 µl of 1 mg/ml IL-22 plus 100mg/ml SNAC murine IL22Fc and 100 mg/ml SNAC through intra-rectal route using 20G flexible plastic tubing. For colon IL-22 level measurement, whole colon was collected 15', 30', 1hr, 2hr, 4hr, or 24hr after dosing. After removing the stool, the colon was cut longitudinally and washed in 45 ml wash buffer (PBS + 0.2% tween 20) for 5 minutes. After repeating the wash 4 times, the colon was put in Safe-Lock tubes containing 1 ml NP40 Cell Lysis buffer (Invitrogen). Colon samples were lysed with TissueLyser II (Qiagen). Supernatant was collected for IL-22 measurement by ELISA (Mouse/rat IL-22 ELISA kit from R&D System). For serum IL-22 level measurement, serum was collected after 15', 30', 1hr, 2hr, 4hr, or 24hr after dosing. IL-22 level was measured by ELISA (Mouse/rat IL-22 ELISA kit from R&D System). Results are shown in Figure 3. Higher levels of colon IL-22 were seen in the Fc-bound IL-22 (Figure 3a), whereas an initial spike in systemic IL-22 levels were seen with IL-22 (Figure 3b).

### Example 3: IL-22 Gut vs Systemic delivery

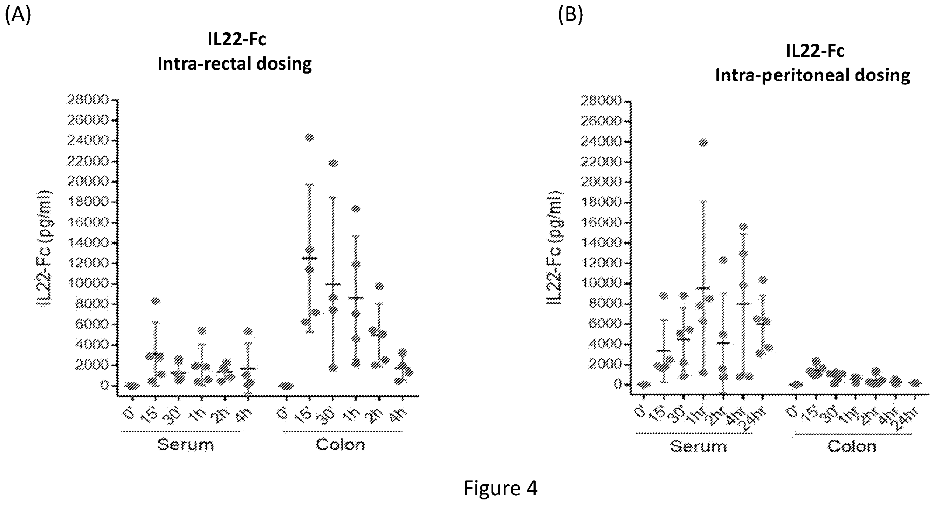

Mice were administered murine IL22Fc either intra-rectally or intra-peritoneally to examiner differences in systemic versus colonic IL-22 levels. C57B1/6 mice were dosed with 78µg murine IL22Fc plus 100 mg/ml SNAC intra-rectally using 20G flexible plastic tubing, or with 3µg murine IL22Fc intra-peritoneally. For colon IL-22 level measurement, whole colon was collected 15', 30', 1hr, 2hr, 4hr or 24hr after dosing. After removing the stool, the colon was cut longitudinally and washed in 45 ml wash buffer (PBS + 0.2% tween 20) for 5 minutes. After repeating the wash 4 times, the colon was put in Safe-Lock tubes containing 1 ml NP40 Cell Lysis buffer (Invitrogen). Colon was lysed with TissueLyser II (Qiagen). Supernatant was collected for IL-22 measurement by ELISA (Mouse/rat IL22 ELISA kit from R&D System). For serum IL-22 level measurement, serum was collected after 15', 30', 1hr, 2hr, 4hr or 24hr after dosing. IL-22 level was measured by ELISA (Mouse/rat IL22 ELISA kit from R&D System). Results are shown in Figure 4. Higher levels of colon signaling were seen in intra-rectal administration (Figure 4a). Higher levels of serum signaling were seen in intra-peritoneal administration (Figure 4b).

### Example 4: Gut delivered IL-22 pharmacodynamics

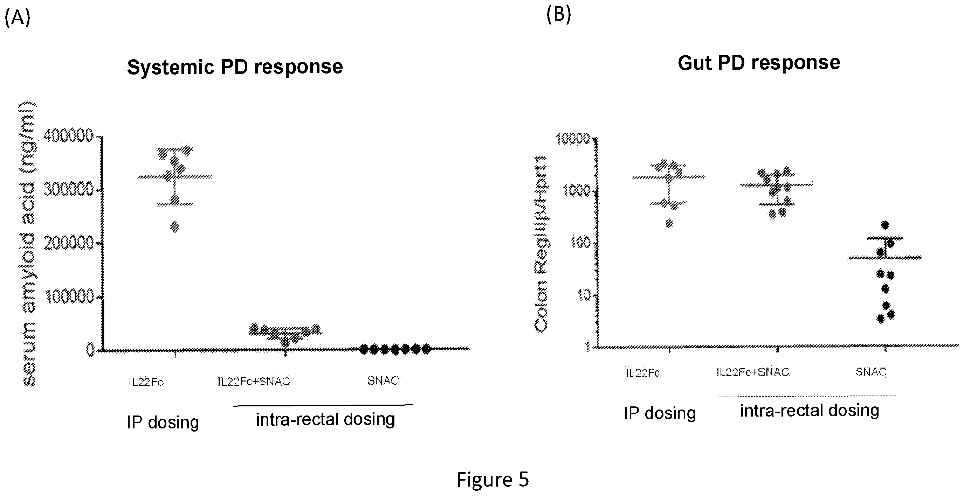

Intra-peritoneal and intra-rectal administration of murine IL22Fc was examined for ability to induce system PD response measured by increase serum amyloid acid levels or gut PD response measured by colon Reg3β expression in the gut. Murine IL22Fc was administered intraperitoneally while murine IL22Fc plus SNAC was administered intra-rectally. SNAC alone was administered as a control. Serum amyloid acid level was measured by ELISA (SAA ELISA kit, R&D System). Colon regIIIβ expression was measured by qPCR. In brief, middle colon was collected and snap frozen in liquid nitrogen. For RNA extraction, colon tissue was put in 0.8ml RTL lyse buffer (Qiagen) in Safe-Lock tubes, and lysed in TissueLyser II. Supernatant was used for RNA purification and qPCR on Quant studio 7. Results are shown in Figure 5. Systemic levels of SAA are shown in Figure 5a and show higher levels of SAA in intra-peritoneally administered murine IL22Fc. Gut levels of SAA are shown in Figure 5b and show high levels of SAA in the gut from both intra-peritoneally and intra-rectal dosing of murine II,22Fc. There is greater selective of local versus systemic pharmacodynamic effect by intra-rectal dosing of murine IL22Fc plus SNAC compared to systemic dosing of murine IL22Fc through intra-perotoneal route.

## Claims

1. An oral, intra-rectal, or other gut-related pharmaceutical composition comprising an IL-22 protein and an absorption enhancer, wherein the absorption enhancer is N(8-[2-hydroxybenzoyl]amino)caprylic acid (NAC) or a salt of said NAC.

2. The oral, intra-rectal, or other gut-related pharmaceutical composition of claim 1, wherein the IL-22 has at least 80% identity to SEQ ID NO:1, 2, 3, or 4.

3. The oral, intra-rectal, or other gut-related pharmaceutical composition of claim 1 or claim 2, wherein the composition is a solid pharmaceutical composition.

4. The oral, intra-rectal, or other gut-related pharmaceutical composition of any one of claims 1-3, wherein said salt of said NAC is selected from the group consisting of a monosodium salt, a disodium salt, and a combination thereof.

5. The oral, intra-rectal, or other gut-related pharmaceutical composition of any one of claims 1-4, further comprising a protease inhibitor, wherein said protease inhibitor is selected from the group consisting of a serpin, a suicide inhibitor, a transition state inhibitor, a protein protease inhibitor, a cheating agent, a cysteine protease inhibitor, a threonine protease inhibitor, an aspartic protease inhibitor, and a metalloprotease inhibitor.

6. The oral, intra-rectal, or other gut-related pharmaceutical composition of any one of claims 1-5, further comprising EDTA or a salt thereof.

7. The oral, intra-rectal, or other gut-related pharmaceutical composition of any one of claims 1-6, further comprising a coating that inhibits digestion of said composition in a stomach of a subject; an enteric coating; or a gelatin coating.

8. The oral, intra-rectal, or other gut-related pharmaceutical composition of any one of claims 1-7, wherein the IL-22 protein is an IL-22 Fc-fusion protein comprising an Fc linked to said IL-22 protein .

9. The oral, intra-rectal, or other gut-related pharmaceutical composition of claim 8, wherein the Fc is a human IgG1 Fc.

10. The oral, intra-rectal, or other gut-related pharmaceutical composition of claim 9, wherein the human IgG1 Fc comprises one or more mutations altering effector function of said Fc, wherein the human IgG1 comprises a substitution at N297.

11. The oral, intra-rectal, or other gut-related pharmaceutical composition of claim 10, wherein the substitution at N297 is N297G.

12. The oral, intra-rectal, or other gut-related pharmaceutical composition of any one of claims 8-11, wherein a linker connects the Fc and human IL-22 portions of said protein.

13. The oral, intra-rectal, or other gut-related pharmaceutical composition of any one of claims 1 to 12, for use in a method for treating an inflammatory disorder.

14. The oral, intra-rectal, or other gut-related pharmaceutical composition for use according to claim 13, wherein the inflammatory disorder is selected from the group consisting of: inflammatory bowel disorder, Crohn's disease, ulcerative colitis, Type II diabetes, Type II diabetes with morbid obesity, wounds (including diabetic wounds and diabetic ulcers), burns, ulcers (including pressure ulcer and venous ulcer), graft versus host disease (GVHD), microbial infection, acute kidney injury, acute pancreatitis, cardiovascular conditions, metabolic syndrome, acute endotoxemia, sepsis, atherosclerosis, cardiovascular disease, metabolic syndrome, endotoxemia (acute and mild), acute coronary heart disease, hypertension, dyslipemia, hyperglycemia, lipid metabolism disorders, hepatitis, acute hepatitis, renal failure, acute renal failure, renal draft failure, post cadaveric renal transplant delayed graft function, contrast induced nephropathy, pancreatitis, liver fibrosis and lung fibrosis.

15. The oral, intra-rectal, or other gut-related pharmaceutical composition for use according to claim 14, wherein the inflammatory disorder is inflammatory bowel disorder, Crohn's disease, or ulcerative colitis.

## Patentansprüche

1. Orale, intrarektale oder andere darmbezogene pharmazeutische Zusammensetzung, umfassend ein IL-22-Protein und einen Absorptionsverstärker, wobei der Absorptionsverstärker N(8-[2-Hydroxybenzoyl]amino)caprylsäure (NAC) oder ein Salz der NAC ist.

2. Orale, intrarektale oder andere darmbezogene pharmazeutische Zusammensetzung nach Anspruch 1, wobei das IL-22 wenigstens 80 % Identität mit SEQ ID NO: 1, 2, 3 oder 4 aufweist.

3. Orale, intrarektale oder andere darmbezogene pharmazeutische Zusammensetzung nach Anspruch 1 oder Anspruch 2, wobei die Zusammensetzung eine feste pharmazeutische Zusammensetzung ist.

4. Orale, intrarektale oder andere darmbezogene pharmazeutische Zusammensetzung nach einem der Ansprüche 1-3, wobei das Salz der NAC ausgewählt ist aus der Gruppe bestehend aus einem Mononatriumsalz, einem Dinatriumsalz und einer Kombination davon.

5. Orale, intrarektale oder andere darmbezogene pharmazeutische Zusammensetzung nach einem der Ansprüche 1-4, ferner umfassend einen Proteaseinhibitor, wobei der Proteaseinhibitor ausgewählt ist aus der Gruppe bestehend aus einem Serpin, einem Suizidinhibitor, einem Übergangszustandinhibitor, einem Proteinproteaseinhibitor, einem Chelatbildner, einem Cysteinproteaseinhibitor, einem Threoninproteaseinhibitor, einem Aspartatproteaseinhibitor und einem Metalloproteaseinhibitor.

6. Orale, intrarektale oder andere darmbezogene pharmazeutische Zusammensetzung nach einem der Ansprüche 1-5, ferner umfassend EDTA oder ein Salz davon.

7. Orale, intrarektale oder andere darmbezogene pharmazeutische Zusammensetzung nach einem der Ansprüche 1-6, ferner umfassend eine Beschichtung, die Verdauung der Zusammensetzung in einem Magen eines Subjekts hemmt, eine enterische Beschichtung oder eine Gelatinebeschichtung.

8. Orale, intrarektale oder andere darmbezogene pharmazeutische Zusammensetzung nach einem der Ansprüche 1-7, wobei das IL-22-Protein ein IL-22-Fc-Fusionsprotein ist, das ein mit dem IL-22-Protein verknüpftes Fc umfasst.

9. Orale, intrarektale oder andere darmbezogene pharmazeutische Zusammensetzung nach Anspruch 8, wobei das Fc ein menschliches IgG1-Fc ist.

10. Orale, intrarektale oder andere darmbezogene pharmazeutische Zusammensetzung nach Anspruch 9, wobei das menschliche IgG1-Fc eine oder mehrere Mutationen umfasst, welche die Effektorfunktion des Fc verändern, wobei das menschliche IgG1 eine Substitution an N297 umfasst.

11. Orale, intrarektale oder andere darmbezogene pharmazeutische Zusammensetzung nach Anspruch 10, wobei es sich bei der Substitution an N297 um N297G handelt.

12. Orale, intrarektale oder andere darmbezogene pharmazeutische Zusammensetzung nach einem der Ansprüche 8-11, wobei ein Linker die Fc- und die menschlichen IL-22-Teile des Proteins verbindet.

13. Orale, intrarektale oder andere darmbezogene pharmazeutische Zusammensetzung nach einem der Ansprüche 1 bis 12, zur Verwendung in einem Verfahren zur Behandlung einer entzündlichen Erkrankung.

14. Orale, intrarektale oder andere darmbezogene pharmazeutische Zusammensetzung zur Verwendung nach Anspruch 13, wobei die entzündliche Erkrankung ausgewählt ist aus der Gruppe bestehend aus: entzündlicher Darmerkrankung, Morbus Crohn, Colitis ulcerosa, Typ-II-Diabetes, Typ-II-Diabetes mit morbider Adipositas, Wunden (einschließlich diabetischer Wunden und diabetischer Geschwüre), Verbrennungen, Geschwüre (einschließlich Druckgeschwüre und venöser Geschwüre), Graft-versus-Host-Krankheit (GvHK), mikrobieller Infektion, akuter Nierenschädigung, akuter Pankreatitis, Herz-Kreislauf-Leiden, metabolischem Syndrom, akuter Endotoxämie, Sepsis, Atherosklerose, Herz-Kreislauf-Erkrankung, metabolischem Syndrom, Endotoxämie (akute und leichte), akuter koronarer Herzkrankheit, Bluthochdruck, Dyslipämie, Hyperglykämie, Fettstoffwechselstörungen, Hepatitis, akuter Hepatitis, Nierenversagen, akutem Nierenversagen, Nierentransplantatversagen, verzögerter Transplantatfunktion nach postmortaler Transplantation, Kontrastmittelnephropathie, Pankreatitis, Leberfibrose und Lungenfibrose.

15. Orale, intrarektale oder andere darmbezogene pharmazeutische Zusammensetzung zur Verwendung nach Anspruch 14, wobei die inflammatorische Erkrankung entzündliche Darmerkrankung, Morbus Crohn oder Colitis ulcerosa ist.

## Revendications

1. Composition pharmaceutique orale, intra-rectale ou autrement liée aux intestins comprenant une protéine IL-22 et un agent d'amélioration de l'absorption, l'agent d'amélioration de l'absorption étant l'acide N(8-[2-hydroxybenzoyl]amino)caprylique (NAC) ou un sel dudit NAC.

2. Composition pharmaceutique orale, intra-rectale ou autrement liée aux intestins selon la revendication 1, l'IL-22 ayant au moins 80 % d'identité avec la SEQ ID NO: 1, 2, 3 ou 4.

3. Composition pharmaceutique orale, intra-rectale ou autrement liée aux intestins selon la revendication 1 ou la revendication 2, la composition étant une composition pharmaceutique solide.

4. Composition pharmaceutique orale, intra-rectale ou autrement liée aux intestins selon l'une quelconque des revendications 1 à 3, ledit sel dudit NAC étant choisi dans le groupe constitué par un sel monosodique, un sel disodique et une combinaison correspondante.

5. Composition pharmaceutique orale, intra-rectale ou autrement liée aux intestins selon l'une quelconque des revendications 1 à 4, comprenant en outre un inhibiteur de protéase, ledit inhibiteur de protéase étant choisi dans le groupe constitué par une serpine, un inhibiteur suicide, un inhibiteur d'état de transition, un inhibiteur de protéase protéique, un agent de triche, un inhibiteur de protéase à cystéine, un inhibiteur de protéase à thréonine, un inhibiteur de protéase aspartique et un inhibiteur de métalloprotéase.

6. Composition pharmaceutique orale, intra-rectale ou autrement liée aux intestins selon l'une quelconque des revendications 1 à 5, comprenant en outre de l'EDTA ou un sel correspondant.

7. Composition pharmaceutique orale, intra-rectale ou autrement liée aux intestins selon l'une quelconque des revendications 1 à 6, comprenant en outre un revêtement qui inhibe la digestion de ladite composition dans un estomac d'un sujet ; un revêtement entérique ; ou un revêtement de gélatine.

8. Composition pharmaceutique orale, intra-rectale ou autrement liée aux intestins selon l'une quelconque des revendications 1 à 7, la protéine IL-22 étant une protéine de fusion Fc IL-22 comprenant un Fc lié à ladite protéine IL-22.

9. Composition pharmaceutique orale, intra-rectale ou autrement liée aux intestins selon la revendication 8, le Fc étant un Fc d'IgG1 humaine.

10. Composition pharmaceutique orale, intra-rectale ou autrement liée aux intestins selon la revendication 9, le Fc d'IgG1 humaine comprenant une ou plusieurs mutations altérant la fonction effectrice dudit Fc, l'IgG1 humaine comprenant une substitution au niveau de N297.

11. Composition pharmaceutique orale, intra-rectale ou autrement liée aux intestins selon la revendication 10, la substitution au niveau de N297 étant N297G.

12. Composition pharmaceutique orale, intra-rectale ou autrement liée aux intestins selon l'une quelconque des revendications 8 à 11, un lieur reliant le Fc et des parties de IL-22 humaine de ladite protéine

13. Composition pharmaceutique orale, intra-rectale ou autrement liée aux intestins selon l'une quelconque des revendications 1 à 12, pour une utilisation dans un procédé pour le traitement d'un trouble inflammatoire.

14. Composition pharmaceutique orale, intra-rectale ou autrement liée aux intestins pour une utilisation selon la revendication 13, le trouble inflammatoire étant choisi dans le groupe constitué par : trouble intestinal inflammatoire, maladie de Crohn, colite ulcéreuse, diabète de type II, diabète de type II avec obésité morbide, plaies (y compris plaies diabétiques et ulcères diabétiques), brûlures, ulcères (y compris escarres et ulcères veineux), réaction du greffon contre l'hôte (GVHD), infection microbienne, lésion rénale aiguë, pancréatite aiguë, affections cardiovasculaires, syndrome métabolique, endotoxémie aiguë, septicémie, athérosclérose, maladie cardiovasculaire, syndrome métabolique, endotoxémie (aiguë et légère), maladie coronarienne aiguë, hypertension, dyslipémie, hyperglycémie, troubles du métabolisme lipidique, hépatite, hépatite aiguë, insuffisance rénale, insuffisance rénale aiguë, défaillance du tirage rénal, retard de la fonction du greffon après transplantation rénale cadavérique, néphropathie induite par le contraste, pancréatite, fibrose hépatique et fibrose pulmonaire.

15. Composition pharmaceutique orale, intra-rectale ou autrement liée aux intestins pour une utilisation selon la revendication 14, le trouble inflammatoire étant un trouble intestinal inflammatoire, la maladie de Crohn ou une colite ulcéreuse.
